Europäisches Patentamt

European Patent Office

Office européen des brevets

(11) Publication number: **0 140 127**
**B1**

## EUROPEAN PATENT SPECIFICATION

(45) Date of publication of patent specification: **30.01.91**

(21) Application number: **84111131.3**

(22) Date of filing: **18.09.84**

(51) Int. Cl.⁵: **C 12 P 21/02,** C 12 N 15/00,
C 07 K 13/00, C 07 K 15/26

(54) Process for the preparation of immune interferon.

(30) Priority: **20.09.83 US 534039**

(43) Date of publication of application:
**08.05.85 Bulletin 85/19**

(45) Publication of the grant of the patent:
**30.01.91 Bulletin 91/05**

(84) Designated Contracting States:
**AT BE CH DE FR GB IT LI LU NL SE**

(56) References cited:
**EP-A-0 077 670**
**EP-A-0 088 540**
**DE-A-2 514 537**

(73) Proprietor: **F. HOFFMANN-LA ROCHE AG**
**Postfach 3255**
**CH-4002 Basel (CH)**

(72) Inventor: **Kung, Hsiang-Fu**
**21 West Lincoln Street**
**Verona, N.J. 07044 (US)**

(74) Representative: **Lederer, Franz, Dr. et al**
**Lederer, Keller & Riederer Patentanwälte Lucile-**
**Grahn-Strasse 22**
**D-8000 München 80 (DE)**

Courier Press, Leamington Spa, England.

# EP 0 140 127 B1

**Description**

The present invention relates to a process for the preparation of immune interferon. More specifically, this invention relates to a method for extracting an intact sequence form of recombinant human immune interferon from a preparation including transformed microorganisms containing this protein. The extraction is performed with a reagent, such as guanidine-HCl, which inhibits protease or enzyme activity but does not affect the activity of the desired protein. The remaining purification procedure steps after guanidine-HCl extraction may be those known to the person skilled in the protein purification art.

Brief Description of the Figures

Figure 1 illustrates sodium dodecyl sulfate-polyacrylamide gel electrophoresis (SDS-PAGE) of purified recombinant human immune interferon under reducing (A; 0.7M β-mercaptoethanol) and non-reducing conditions (B).

Figure 2 illustrates the comparison between the predicted amino acid sequence of recombinant human immune interferon and the actual DNA sequence of the 15K and 18K rIFN-γ species.

Figure 3 illustrates the HPLC separation of C-terminal peptides of the 15K and 18K rIFN-γ after CNBr treatment.

The prior art has devised various methods for effecting the extraction and purification of the family of anti-viral proteins known as interferon. To date there are three known major classes of interferon: IFN-α (leukocyte), IFN-β (fibroblast) and IFN-γ (immune). Although the various interferon classes may be related in terms of antiviral, anti-proliferative or structural arrangement, the prior art has had so far been unable to devise a uniform method for the extraction and purification of all of these classes. Indeed, many of the processes useful for the extraction and purification of leukocyte interferon from a crude mixture of other proteins and cell debris would work to extract and purify fibroblast or immune interferon from the same kind of preparation.

The extraction step in the purification processes of the prior art typically involved either the mechanical (i.e. sonification) or chemical lysis of microorganisms which have produced, often through recombinant techniques, the desired "foreign" protein. However, during this mechanical or chemical lysis procedure various cellular proteases are also released into the mixture. These proteases are then free to enzymatically act upon and degrade the "foreign" protein in the mixture. These proteases can, therefore, hinder or inhibit the purification to homogeneity of the complete or mature and biologically active form of the "foreign" protein.

It is, therefore, an object of the present invention to provide a method which overcomes the limiations of the prior art extraction techniques whereby the intact sequence form of immune interferon is obtained and whereby fragments from proteolytic degradation are eliminated from the purified immune interferon preparations.

Description of the Preferred Embodiments

It has been discovered in the case of immune interferon, that extractioon with any of the conventional techniques such as sonification or mechanical lysis described in the prior art will not yield immune interferon with an intact or complete amino acid sequence. Only recently has recombinant technology advanced to the point at which it is possible to obtain sufficient quantities of rINF-γ so as to characterize it and determine its amino acid sequence. When conventional prior art techniques were utilized to extract rINF-γ preparations and the amino acid sequence of purified material was determined, it was discovered that purified preparation was, in fact, comprised of a variety of related protein species of different molecular weight. It has further been discovered by amino acid sequencing that these protein species are actually the intact sequence form of immune interferon in combination with proteolytic fragments of the intact sequence protein. Therefore, the prior art has yet been unable to extract and purify to homogeneity the intact form of immune interferon free of other proteolytic fragments of immune interferon.

It has been discovered that the degradation of rIFN-γ can be prevented by use of a reagent, such as quanidine-HCl, which inhibits protease or enzyme activity and which does not affect the activty of the rIFN-γ, in the initial extraction step of a purification procedure so as to obtain homogeneous and intact molecules. Sonification of frozen cells in the absence of guanidine-HCl yielded mainly a proteolytic product (15K rIFN-γ) with missing C-terminal amino acid residue Nos.132—146 of IFN-γ. Amino acid composition and N-terminal sequence of the intact molecule was consistent with that expected from the DNA sequence. The DNA base sequence of rIFN-γ as used throughout this specification is as described in reference 1.

It has surprisingly been found that immune interferon retains its biological activity after a guanidine-HCl extraction step even though guanidine-HCl destroys the biological activity when added to a purified preparation of immune interferon. It is preferred that in the practice of this invention the transformed microorganisms be suspended in the guanidine-HCl.

The anti-proteolytic agent of this invention may be any guanidinium salt. Among such guanidinium salts there are salts with organic acids such as for example acetic acid or mineral acids such as for example the hydrohalides, i.e., hydrobromide, hydrochloride, hydrofluoride and hydroiodide. The preferred guanidinium salt is guanidine hydrochloride. The concentration of guanidinium salt in the treatment of the microorganisms is not critical in that any effective amount may be used. It is preferred, however, that a 3 to

2

7M solution of the guanidinium salt be used for treating the microorganisms and that about 3 to 9 volumes of the salt solution per gram of transformed microorganisms be used. The concentration of the salt may be achieved by making the salt up in a solvent such as water or aqueous buffers, such as ammonium acetate, pyridine/acetic acid, and the like. It is also foreseeable that in the practice of this invention other protease inhibitor reagents may be used, such as urea or thiocyanate.

Purified rIFN-γ can be finally obtained by applying the E. coli supernatant after extraction and appropriate dilution directly on a purification means, preferably using a monoclonal antibody purification step. The extraction and purification process can be automated for large-scale production. Thus the invention makes possible for the first time the availability of large amounts of homogeneous rIFN-γ and thus will permit extensive clinical trials, biological studies, X-ray crystallography and structure-function studies.

Further preferred embodiments will be illustrated in the following specification and examples.

Recombinant human immune interferon produced in E. coli is preferably extracted from frozen cell paste by 7M guanidine-HCl and purified by suitable purification means such as through monoclonal antibody affinity columns. A purified interferon with an apparent M.W. of about 18,000 daltons (18K) by SDS-PAGE has been obtained by guandine extraction whereas a lower M.W. species (major species of about 15,000 daltons, 15K) was isolated by sonification in the absence of guanidine. The amino terminal sequence of both the 18K and 15K proteins were consistent with the sequence predicted from the DNA sequence for this human immune interferon protein.

The C-terminal sequence was determined by analyzing and sequencing purified C-terminal peptide after CNBr treatment. The amino acid composition and sequence of the C-terminal peptide released from the 18K rIFN-γ matched with the predicted sequence of rIFN-γ indicating that the 18K species is the intact molecule. On the other hand, upon CNBr treatment a different peptide fragment was released from the C-terminal of the 15K rIFN-γ. Based on amino acid and sequence analysis, this different peptide corresponded to amino acid residues Nos. 121—131 indicating that the 15K species was a proteolytic product.

In a preferred embodiment of this invention, the microorganisms employed as the recipient in the fermentation procedures and unless otherwise noted, is the microorganism Escherichia coli K-12 strain 294 as described in British Patent Publication No. 2055382A which has been deposited with the American Type Culture Collection under ATCC Accession No. 31446 on October 28, 1978. Furthermore, all recombinant DNA work herein was performed in compliance with applicable guidelines of the National Institutes of Health.

The invention, however, includes the use not only of E. coli K-12 strain 294, mentioned above, but also of other known E. coli strains such as E. coli MA210 of E. coli RR1 (ATCC No. 31343), or other microorganisms many of which are publicly available or are deposited and available from recognized microorganism depository institutions, such as the American Type Culture Collection (see e.g. the ATCC catalogue).

Monoclonal antibodies were made against a synthetic polypeptide consisting of the last 16 amino acid residues of the C-terminus of rIFN-γ, i.e. amino acid residues Nos. 131—146. One of the monoclonal anti-bodies (Mo γ 2—11.1) was used for the purification of rIFN-γ. More specifically, the monoclonal antibodies and the antibody affinity column of this invention were prepared as described in European patent application publication No. 103898.

The invention is further illustrated by the following examples.

## Example 1

Synthesis of carrier protein-polypeptide complex used as anitgen

The polypeptide H-Lys-Arg-Lys-Arg-Ser-Gln-Met-Leu-Phe-Arg-Gly-Arg-Arg-Ala-Ser-Gln-OH was coupled with thyroglobulin (hereinafter TG) according to the method of Goodfriend et al., Science *144*, 1334 (1964).

The peptide itself can be produced by conventional methods of peptide synthesis. Either of the solid phase and liquid phase method may be used, although the liquid phase synthetic method is advantageous in many cases. Such methods of peptide synthesis are described. For example, by Schröder and Lübke in "The Peptides", Vol. 1, Academic Press, New York, USA., 1966, or by Izumiya et al. in "Peptide Syntheses", Maruzen, Tokyo, Japan, 1975 or by Haruaki Yajima in "Experiments in Biochemistry", Vol. 1, pages 207—400, Tokyo Kagaku Dojin, 1977, and include, among others, the azide method, chloride method, acid anhydride method, mixed acid anhydride method, DCC method, active ester method, method using Wood-ward reagent K, carbodiimidazole method, oxidation-reduction method and DCC/additive (e.g. HONB, HOBt, HOSu) method.

For a detailed description of the preparation of the 131—146 fragment of rIFN-γ see published European patent application No. 103 898.

2.5 mg of said polypeptide were mixed with 3.75 mg of TG and, following addition of 2 ml of 50 mM phosphate buffer, the mixture was stirred well in ice water. Thereto was gradually added drop by drop a solution of 30.4 mg of carbodiimide hydrochloride in 200 ml of distilled water. Thereafter, the mixture was stirred in ice water for 3 hours. After completion of the reaction, dialysis was performed against distilled water to a sufficient extent, followed by lyophilization to give 4.7 mg of a protein complex.

Example 2

Preparation of enzyme-linked antigen for Enzyme Immunoassay (EIA) for antibody detection

The enzyme-linked antigen for EIA was prepared according to Kitagawa et al., Journal of Biochemistry *79*, 233 (1976).

(i) Introduction of a maleimido group into the polypeptide

The polypeptide (350 nmoles) as obtained in Example 1 was dissolved in 1 ml of 100 mM phosphate buffer (pH 6.8), and the solution was added to a solution of 585 μg (1.75 μmoles) of N-(4-carboxycyclohexyl-methyl)maleimide N-hydroxy-succinimide ester in 70 μl of N,N-dimethylformamide. The mixture was stirred at 30°C for 30 minutes. After completion of the reaction, fractionation was performed using a Sephadex G-25 column to give 185 nmoles of a polypeptide fraction with the maleimido group introduced therein.

(ii) Coupling of the maleimido-group-containing polypeptide with β-D-galactosidase

The maleimido-containing polypeptide (16.5 nmoles) was mixed with 3.3 nmoles of β-D-galactosidase. After 18 hours of reaction at 4°C, 412.5 nmoles of β-mercaptoethanol were added to terminate the reaction. The β-D-galactosidase-coupled polypeptide was fractionated on a Sepharose 6B column and used for the subsequent experiments.

Example 3

Immunization

Each of 6 female BALB/C mice aged 7 to 8 weeks was subcutaneously inoculated with 40 μg (on the basis of the protein) of the protein complex obtained in Example 1 as anitgen in intimate admixture with Freund's complete adjuvant (primary immunization). Two weeks after the primary immunization, the mice were again subcutaneously inoculated with the antigen at the same dose as above in intimate admixture with Freund's incomplete adjuvant (secondary immunization). Further two weeks later, a third immunization was made in the same manner as in the secondary immunization. Six days after the third imunization, partial blood sampling was made from the mice and the serum antibody titers were determined by the EIA method described below (compare Immunopharmacology *1*, 3 [1978]). The mouse numbered γ-2 gave the highest antibody titer and was subjected to a final immunization by intravenous inoculation with 120 μg of the antigen dissolved in 0.5 ml of aqueous sodium chloride. The antibody titer data for each mouse are shown in Table 1.

# EP 0 140 127 B1

## Table 1

## Antipeptide antibody titers in immunized mice

| | | B/T (%) | |
|---|---|---|---|
| Mouse No. | Primary immu-nization 1) | Secondary immunization 2) | Third immu-nization 3) |
| γ-1 | - 4) | N.D. | 24.5 |
| 2 | N.D. 5) | 19.3 | 35.3 |
| 3 | - | N.D. | 24.7 |
| 4 | N.D. | 1.3 | 1.7 |
| 5 | N.D. | 1.8 | 5.0 |
| 6 | - | N.D. | 0.8 |
| Normal-mouse | 0.6 | 0.1 | N.D. |

1) Serum dilution ratio: 1/1000

2) Serum dilution ratio: 1/6300

3) Serum dilution ratio: 1/7800

4) -: Not detectable

5) N.D.: Not determined

B/T: (Bound enzyme activity/total added enzyme activity) x 100

EIA method

The detection of antibody activity in the serum of mice immunized with the protein complex obtained in Example 2 or in the hybridoma supernatant was conducted by the EIA method [Immunology 1, 3 (1978)]. Thus, the serum or hybridoma supernatant was diluted with buffer A (20 mM $Na_2HPO_4$, 100 mM NaCl, 0.1% $NaN_3$, 1 mM $MgCl_2$, pH 7.0), a 100-μl portion of the dilution was mixed with 100 μl of the enzyme-linked polypeptide derivative (Example 2) and the reaction was followed to proceed at 24°C for 24 hours. Thereafter, 100 μl of 3% cellulose coupled with rabbit and anit-mouse IgG was added, and the reaction was allowed to proceed at 24°C for 4 hours. After completion of the reaction, the cellulose was washed well with buffer A containing 0.5% of Tween 20, then 500 μl of 20 μg/ml 4-methylumbelliferyl-β-D-galactoside was added and, after 2 hours of reaction at 37°C, 3 ml of 100 mM carbonate buffer (pH 10.5) were added to terminate the reaction. The fluorescence intensity was measured with a fluorometer (excitation: 365 nm; emission: 450 nm).

## Example 4

Cell fusion

Three days after the final immunization as described in Example 3, the spleen was excised from the γ-2 mouse, filtered under pressure through a stainless mesh, and suspended in Eagle's minimum essential medium (MEM) to give a spleen cell suspension. For cell fusion, BALB/C mouse-derived P3-x63.Ag8.UI

(P3UI) myeloma cells were used [Current Topics in Microbiology and Immunology, *81*, 1 (1978)]. Cell fusion was performed by the original method of Köhler and Milstein, Nature *256*, 495—497 (1975). Thus, spleen cells and P3UI cells were separately washed three times with serum-free MEM and mixed at a ratio of 5:1 (in number of cells). The mixture was centrifuged at 800 rpm for 15 minutes, whereby the cells were settled. After thorough removal of the supernatant, the sediment was lightly loosened, 0.3 ml of 45% polyethylene glycol (PEG) 6000 (Koch-Light) was added, and the mixture was allowed to stand in a warm water tank maintained at 37°C for 7 minutes so as to effect cell fusion. Thereafter, MEM was added thereto at a rate of 2 ml per minute. After addition of 12 ml in total of MEM, the resulting mixture was centrifuged at 600 rpm for 15 minutes, followed by removal of the supernatant. The cell sediment was suspended in RPMI-1640 medium supplemented with 10% fetal calf serum (RPMI1640-10FCS) in a concentration of $2 \times 10^5$ P3UI cells/ml and each of 144 wells on 24-well multidishes (Linbro) was seeded with 1 ml of the suspension. After seeding, the cells were incubated at 37°C in a 5% carbon dioxide gas incubator. After 24 hours, HAT-selective culture was started by adding RPMI1640—10FCS medium supplemented with HAT ($1 \times 10^{-4}$ M hypoxanthine, $4 \times 10^{-7}$ aminopterin, $1.6 \times 10^{-5}$ M thymidine) medium in an amount of 1 ml per well. The HAT-selective culture was continued while 1 ml of the old medium was replaced by 1 ml of HAT medium 3, 5 and 7 days after start of the culture. The growth of hybridomas was noted 10 to 14 days after cell fusion. When the culture broth turned yellow (about $1 \times 10^6$ cells/ml), the supernatant was collected and examined for the presence of antibody by the EIA method. In this manner, supernatants from 141 wells in which hybridoma growth had been noted were examined. Two wells (γ 2-11 and γ 2-100) showed intense antibody activity and other two wells (γ 2-62 and γ 2-70) presented weak antibody activity.

Example 5

Cloning

Hybridomas from 3 wells (γ 2-11, γ 2-62 and γ 2-100) which were postitive in antibody activity were cloned by the limiting dilution method. Thus, hybridoma cells were suspended in RPMI1640-20FCS in a concentration of at least 2 hydridoma cells/ml and the suspension was distributed in 0.1 ml portions into the wells on a 96-well microplate. In said distribution, $5 \times 10^5$ per well of BALB/C mouse thymocytes were added as feeder cells. As a result, cell proliferation was observed in about 2 weeks. The supernatant was then collected and examined for the presence of antibodies by the EIA method as described in Example 3. Antibody activity was noted in 8 of 19 clones from γ 2-11 well, in 3 of 54 clones from γ 2-62 well, and in 5 of 47 clones from γ 2-100 well (Table 2).

## EP 0 140 127 B1

### Table 2

### Anti-peptide antibody activity of cloned hybridomas

| Hybridoma No. | | B/T (%) |
|---|---|---|
| γ 2-11 | | |
| | 1 | 68 |
| | 2 | 31 |
| | 3 | 63 |
| | 6 | 68 |
| | 7 | 67 |
| | 9 | 69 |
| | 12 | 42 |
| | 18 | 60 |
| γ 2-62 | | |
| | 14 | 20 |
| | 16 | 21 |
| | 34 | 16 |
| γ 2-100 | | |
| | 2 | 69 |
| | 3 | 70 |
| | 16 | 56 |
| | 25 | 80 |
| | 46 | 33 |
| Hyperimmune mouse serum | | 35 |

Example 4

Binding capacity of monoclonal antibodies to IFN-γ

The binding capacity of monoclonal antibodies to IFN-γ was determined by the following method. To 300 μl of a 3% solution of cellulose coupled with rabbit anti-mouse IgG antibody, 300 μl of the culture supernatant of each of 2 or 3 cloned cell lines from each of γ 2-11, γ 2-62 and γ 2-100 wells was added, and the reaction was allowed to proceed at room temperature for 12 to 20 hours. Then, the cellulose was thoroughly washed with physiological saline, and 550 U/ml of IFN-γ obtained by the procedure mentioned below was added thereto. After 3 to 4 hours of reaction, the supernatant was collected and the IFN-γ obtained by the procedure mentioned below was added thereto. After 3 to 4 hours of reaction, the supernatant was collected and the IFN-γ activity therein was determined by the cytopathic effect (CPE) reading method using a microplate [Applied Microbiology 16, 1706 (1968)]. Thus, 50 μl of MEM was placed

7

in each well of a 96-well microplate and 50 µl of the IFN sample was added to the first well, followed by serial two-fold dilution. To each well thus prepared, 50 µl of a WISH cell suspension (4 × 10$^5$ cells/ml) in 20% FCS-containing MEM was added, and incubation was conducted in a carbon dioxide gas incubator at 37°C for 24 hours. Thereafter, 50 µl of a vesicular stomatitis virus (New Jersey strain) preparation adjusted to a concentration of 2000TCID$_{50}$ (TCID$_{50}$: median tissue culture infecting dose) was added to each well and incubation was performed in a carbon dioxide incubator at 37°C. About 35 hours later, when cells in the IFN sample-free well showed 100% CPE, each well was microscopically observed for the estimation of CPE, and the reciprocal of the dilution factor for the IFN sample in that well in which 50% CPE was noted was referred to as the IFN titer.

The IFN-γ sample used was the supernatant collected 72 hours after stimulation of human peripheral lymphocytes with 40 µg/ml of concanavalin A and 15 ng/ml of 12-O-tetra-decanoylphorbol-13-acetate. Each ml of this culture supernatant contained 4400 units of human IFN-γ (heat and acid labile). If antibodies having binding capacity to IFN-γ are present in the cloned cell culture supernatant, then the added IFN-γ should bind to the antibodies on cellulose and reduction in IFN-γ activity of the supernatant should occur. As a result, for the clone γ 2-11, relatively intense binding activity to IFN-γ was noted and 50—75% of the added IFN-γ (559 U/ml) was bound to antibodies (Table 3).

## Table 3

## Absorption of IFN-γ activity by monoclonal antibodies

| Hybridoma culture | Residual IFN activity (U/ml) | |
|---|---|---|
| | Experiment 1 | Experiment 2 |
| γ 2-11.1 | 138 | 275 |
| γ 2-11.2 | 207 | N.D. |
| γ 2-11.6 | N.D. | 275 |
| γ 2-62.2 | 275 | 550 |
| γ 2-62.3 | 275 | 550 |
| γ 2-100.2 | 550 | N.D. |
| γ 2-100.3 | 550 | N.D. |
| – | 550 | 550 |

### Example 7
Ascites formation by monoclonal antibody-producing hybridomas

Ascites formation was caused by intrapertioneal inoculation of BALB/c mice intraperitoneally pretreated with 0.5 ml of mineral oil with 1 × 10$^6$ γ 2-11.1 clone cells capable of producing antibodies having IFN-γ-binding activity. Ten days after intrapertioneal administration of hybridomas, the ascitic fluid was taken and examined for antibody activity up to 10$^7$-fold dilution. While the antibody activity of the corresponding clone cell culture supernatant was detected up to 10$^4$-fold dilution, the formation of ascites (ascitization) led to an about 1000 times increase in antibody activity.

### Example 8
Monoclonal anitbody purification

Using 4 ml of the ascitic fluid obtained in Example 7 as the starting material, monoclonal antibody purification was performed by the method of Staehelin et al. (Journal of Biological Chemistry *256*, 9750, [1981]). Thus, the ascitic fluid was first centrifuged at 10,000 rpm for 15 minutes to remove fibrin-like substances therefrom and was then diluted with phosphate buffer-saline (PBS: 8.1 mM NaH$_2$PO$_4$, 1.5 mM KH$_2$PO$_4$, 2.7 mM KCl, 137 mM NaCl; pH 7.2) to a concentration at which the ultraviolet absorption at 280 nm

EP 0 140 127 B1

($A_{280}$) would range from 12 to 14. Thereafter, saturated aqueous ammonium sulfate was added to the diluted sample to a sulfate concentration of 47%. The mixture was stirred at 4°C for 60 minutes to effect salting out and was then centrifuged (10,000 rpm, 15 minutes) to give a precipitate. The precipitate was dissolved in 20 mM Tris buffer (pH 7.9) containing 50 mM NaCl and dialyzed against 2 liters of the same buffer. Two hours later, the dialyzing solution was replaced by a fresh 2-liter portion of the same solution and the dialysis was continued for further 15 hours. Thereafter, the precipitate was removed by centrifugation at 10,000 rpm for 15 minutes, and the supernatant was adjusted to a concentration such that the $A_{280}$ value became 20—30. This sample was subjected to fractionation on a DEAE-cellulose column (8 ml, Whatman $DE_{52}$) equilibrated with a sufficient amount of Tris buffer (pH 7.9) containing 50 mM NaCl, elution being made with Tris buffer containing 50 mM NaCl at a flow rate of 1.5 ml/minutes. Under these conditions, the antibody activity was detected mainly in effluent fractions. Confirmation that the purified sample is antibody was made by the SDS-PAGE method as described by Laemmli et al., Nature *227*, 680 (1970). Thus, some of the fractions obtained by ammonium sulfate salting out and DEAE-cellulose fractionation were each subjected to reduction with 2-mercaptoethanol, followed by 17% SDS gel electrophoresis at 30 volts for 24 hours. In agreement with the antibody activity peaks, two bands were noted at positions corresponding to molecular weights of about 55 kilodaltons (H chain) and about 28 kilodaltons (L chain). The thus-purified antibody fraction 17 as examined for IFN-γ-binding by adding IFN-γ (2200 U/ml). It was thus found that about 50% of IFN-γ was bound to the antibody (Table 4).

Table 4

| Sample | Dilution | Residual IFN activity (U/ml) |
|---|---|---|
| γ 2-11.1 fraction 17 | $10^{-1}$ | 1100 |
| | $10^{-2}$ | 1100 |
| | $10^{-3}$ | 2200 |
| | $10^{-4}$ | 2200 |
| Anti-IgE monoclonal antibody | $10^{-1}$ | 2200 |
| | $10^{-2}$ | 2200 |
| | $10^{-3}$ | 2200 |
| | $10^{-4}$ | 2200 |

Example 9

Subclass to which monoclonal antibodies belong

Purified antibody fraction 17 (Example 8) was diluted 10 times and subjected to immuno-precipitation reaction in agar (Ouchterlony test: Immunological Methods, Gel-Diffusion Techniques, Blackwell, Oxford, 1964) using goat anti-mouse IgG1, G2a, G2b and G3 antibodies (Miles) so that the IgG subclass to which γ 2-11.1 monoclonal antibodies might belong could be identified. A single distinct band was found between the monoclonal antibody and the goat anti-mouse IgG2b antibody, while no band formation was noted between the monoclonal antibody and other anti-antibodies. Accordingly, said monoclonal antibody was found to belong to IgG2b (Table 5).

## Table 5

## Monoclonal antibody subclass

| Antigen | Antibody | Precipitation curve |
|---|---|---|
| Monoclonal antibody of the present invention (fraction 17) | Anti-IgG1 | - |
| " | Anti-IgG2a | - |
| " | Anti-IgG2b | + |
| " | Anti-IgG3 | - |

### Example 10

Twenty-five ml (65.3 mg) of the monoclonal antibody from the effluent fractions as purified by the procedure of Example 8 was dialyzed overnight against 0.1M NaHCO₃ (pH 8.3). Separately, 25 ml of AFFI-GEL 10 (Bio-Rad) was thoroughly washed with water using a glass filter, suspended in 0.1M NaHCO₃ (pH 8.3) and mixed with the above antibody. The mixture was stirred gently at 4°C for 4 hours to effect the reaction, and then allowed to stand at 4°C overnight. The AFFI-GEL 10 was washed well with 0.1M NaHCO₃ (pH 8.3) using a glass filter. To the gel were added 25 ml of a solution (pH 8.0) containing 0.1M ethanolamine and 0.15M NaCl. The mixture was shaken at 4°C for an hour so as to block possibly remaining unreacted active groups. Then, the gel was washed well with PBS, and suspended in 25 ml of 0.1% NaN₃-containing PBS. The suspension was stored at 4°C. Based on the amount of the added antibody and the amount of the antibody in the recovered filtrate, it was found that the antibody was conjugated to the gel in a proportion of 2.35 mg/ml of gel. A column was packed with the reaction product obtained in this manner and used as an antibody column.

### Example 11

E. coli RR1 (pRK148cl$_{ts}$, pRC231/IFI-900) (construction of this recombinant organism is described in detail in European patent application publication No. 99084) was used for rIFN-γ production. Plasmids pRK248cl$_{ts}$ and pRC231/IFI-900 contained temperature sensitive Lambda repressopr and IFN-γ genes respectively. Expression of rIFN-γ gene was under the control of the P$_L$ promoter.

Overnight cultures of E. coli RR1 (pRK248cl$_{ts}$, pRC231/IFI-900) were grown in LB broth at 30°C. One liter of the overnight culture was diluted to 10 liters with minimal M-9 medium containing casamino acids. At logarithmic growth, the culture was shifted from 30° to 42°C and contiued to grow at 42°C for 2—3 hours. Bacteria were harvested by centrifugation and the bacterial pellets were stored at −20°C until used. All fermentations and procedures were performed in accordance with recombinant DNA guidelines of the National Institutes of Health.

Monoclonal antibodies were made against synthetic 131—146 C-terminal peptide of rIFN-γ in the manner described above. Monoclonal antibody Mo γ 2-11.1 was used for the purification of rIFN-γ. The monoclonal antibody column was prepared as described in Example 10.

Carboxymethylation of rIFN-γ by ¹⁴C-iodoacetic acid was carried out in the presence of 6M guanidine-HCl as described in reference (3). Excess reagent was removed by HPLC on C8 reverse-phase column. Carboxypeptidase digestion was performed in 0.2M NH₄HCO₃ as described in reference (4).

The rIFN-γ was treated with CNBr (100-fold molar excess over methionine) in 70 percent formic acid as described in reference (5). CNBr peptides were separated by HPLC on C-18 reverse-phase column. A linear gradient of 0 to 70 percent of CH₃CN in 0.1% trifluoroacetic acid was used for peptide elution.

Protein or peptide samples were hydrolyzed for 20—24 hours in sealed, N₂-flushed, evacuated tubes in constant boiling HCl containing 4% thioglycolic acid. Amino acid analyses were performed using a fluorescamine amino acid analyzer as described in reference (6).

An ABI (Applied Biosystem, Inc.) gas-phase sequencer 470A was used for sequence analyses of carboxymethylated proteins as described in reference (7). Samples of PTH-amino acids were identified by reverse-phase HPLC on an ultrasphere ODS column as described in reference (8).

All purification steps were carried out at 4°C. Frozen cells (25 g) were suspended in three volumes (75 ml) of 7M guanidine-HCl (pH 7). The mixture was stirred for 1 h and then centrifuged for 1 h at 30,000 × g. The supernatant was diluted 10-fold with Dulbecco's phosphate buffered saline (PBS) or 0.15M sodium borate buffer (pH 9.5) and then centrifuged for 30 min. at 30,000 × g. Alternatively, frozen cells (25 g) were suspended in 1.5 volumes (37.5 ml) of 0.15 M sodium borate buffer (pH 9.5) and stirred for 1 h. The mixture was sonicated 5 times for 30 seconds and then centrifuged for 1 h at 30,000 × g. The supernatants from either guanidine-HCl extraction or sonification were mixed for 1 h on a rotating shaker with 25 ml silica and pre-washed with PBS. The mixture was poured onto a column and the column was washed with 20—30 column volumes of 1M NaCl. The column was then eluted with 0.5M tetramethyl-ammonium chloride in 0.01M sodium borate buffer (pH 8.0). Interferon activity was eluted in about 200 ml and separated into 4 pools. Each pool was loaded onto a monoclonal antibody (γ 2-11.1) affinity column (4 ml bed volume) equilibrated with PBS. After washing with 10 column volumes of PBS buffer, the column was eluted with either 1M guanidine-HCl or 50% ethylene gylcol containing 1M NaCl and 20 mM sodium phosphate buffer (pH 7.0). Interferon activity was eluted in the first 20 ml.

SDS-PAGE was performed as described by Laemmli (reference 9). Protein was determined by fluorescamine analysis with cyrstalline bovine serum albumin as the reference standard. Interferon activity was determined by a cytopathic effect inhibition assay with vesicular stomatitis virus and human WISH cells as reported in reference 10. All interferon titers are expressed in reference units/ml calibrated against the reference standard of partially purified human immune interfereon.

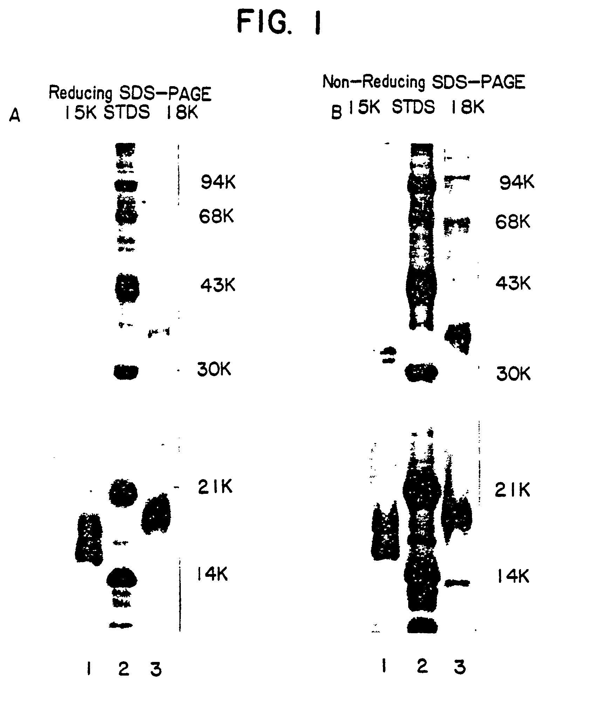

A summary of the extraction purification procedure is presented in Table 6 (page 23). The overall recovery was 25—32% and the purification was about 100 to 769 fold with an average specific activity of $1 \times 10^7$ units/mg. Total yield of rIFN-γ was 3—4 times higher with guanidine extraction. SDS-PAGE of the last stage of purification is shown in Figure 1. The material purified from guanidine extraction showed a single band at about 18,000 daltons (18K rIFN-γ) whereas the material from the sonification procedure yielded a major band at about 15,000 daltons (15K rIFN-γ) and a minor band at about 17,000 daltons (Fig. 1A). On non-reducing gel, dimers and oligomers of rIFN-γ were formed (Fig. 1B).

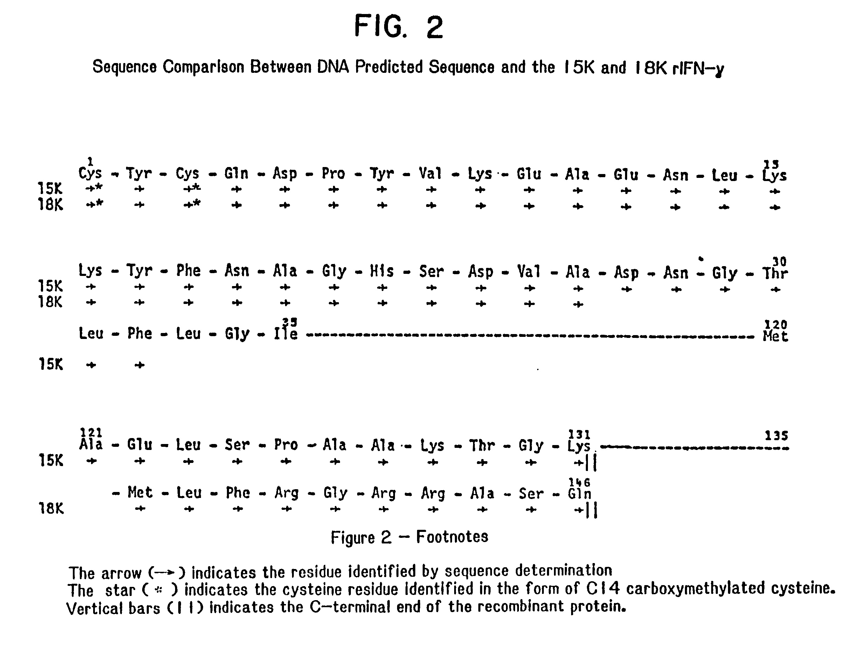

The 18K rIFN-γ was homogeneous and the amino acid composition was consistent with that predicted from the DNA sequence. Amino acid compositions of the 15K and 18K rIFN-γ are given in Table 7. Several hundred picomoles of reduced and carboxymethylated 15K and 18K protein underwent Edman degradation in an automatic gas-phase protein/peptide sequencer. The amino acid N-terminal sequences of the first 32 and 26 residues of the 15K and 18K proteins respectively were in accord with that predicted by the DNA sequence (Fig. 2). $^{14}$C-Carboxymethylated cysteines were detected in the first and third cycles of sequence analyses. No N-terminal methionine was detected. N-termial sequence analysis of the 18K and 15K rIFN-γ demonstrated that the sequence of this area of both proteins is identical to that predicted from the DNA sequence. The C-terminal peptides have also been characterized to determine whether any deletions or changes occured in this region. Amino acid analysis of carboxypeptidase A (CPA) digestion mixture indicated that serine and/or glutamine (C-terminal amino acids) were released from the 18K rIFN-γ, whereas the 15K rIFN-γ was not digested by CPA under same conditions. Since Ser-Gln is the C-terminal sequence of rIFN-γ the 18K species appeared to have the intact C-terminus while the 15K species has a different C-terminal residue (Lys) which is not cleaved by CPA.

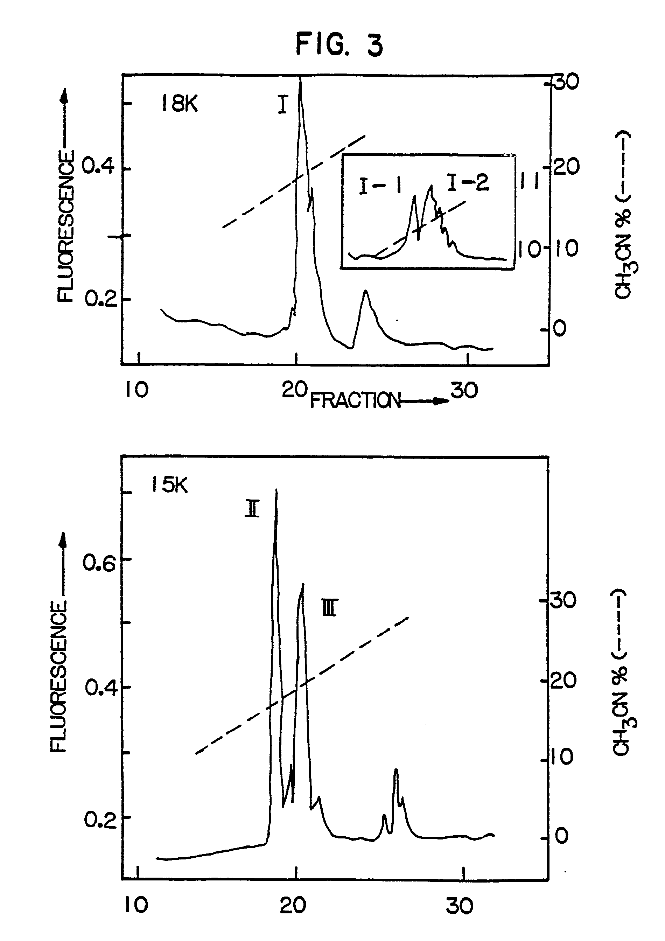

The C-terminal residues predicted from the DNA sequences were further confirmed by analyzing and sequencing the C-terminal peptides after CNBr treatment. C-terminal peptides were separated on the HPLC C-18 reverse-phase column (Fig. 3). A sharp peptide peak (peak II), eluted from the early part of the gradient, was obtained from the 15K rIFN-γ and this peptide was absent from the CNBr digestion mixture of the 18K rIFN-γ. Amino acid analysis of this peptide indicated that this peptide has no homoserine or homoserine lactone and therefore must be the C-terminal CNBr peptide of the 15K protein. Based on amino acid analysis (Table 8), this peptide corresponded to amino acid residues No. 121—131 of IFN-γ (no arginine was detectable). The sequence of the 11 amino acids was confirmed by sequence analysis (Fig. 2). In the case of 18K rIFN-γ, a relatively broad peak was obtained in the early part of the elution. This peak was further separated into two peaks by a shallow gradient. The amino acid analyses indicated that the first peak is the CNBr C-terminal peptide of the 18K protein (Table 8) and the amino acid composition corresponds to amino acid residues No. 138-146. The sequence of the 9 amino acids was verified by sequence determination (Fig. 2). The C-terminal amino acid of the 15K protein was determined to be lysine.

These results indicate that the 18K species was the intact rIFN-γ molecule, whereas the 15K species was a proteolytic product. The peptide bond between amino acid residues No. 131 and No. 132 (Lys-Arg) was cleaved on the 15K species.

## Table 6

### Purification of rIFN-γ

| Purification Step | Total Protein mg | Total Activity units | Specific Activity unit/mg | Purifi- cation -fold | Yield % |
|---|---|---|---|---|---|
| I. Guanidine extraction | | | | | |
| Supernatant | 2.806 | $2.5 \times 10^8$ | $9 \times 10^4$ | – | 100 |
| Silica | 98 | $1.0 \times 10^8$ | $1 \times 10^6$ | 11 | 40 |
| Monoclonal Antibody | 8 | $0.8 \times 10^8$ | $1 \times 10^7$ | 110 | 32 |
| II. Sonification | | | | | |
| Supernatant | 6.136 | $8.0 \times 10^7$ | $1.3 \times 10^4$ | – | 100 |
| Silica | 87 | $4.5 \times 10^7$ | $5.2 \times 10^6$ | 400 | 56 |
| Monoclonal Antibody | 2 | $2.0 \times 10^7$ | $1.0 \times 10^7$ | 769 | 25 |

## Table 7

### Amino Acid Compositions of 15K and 18K rIFN-γ
### (Residue numbers)

|     | 18K (1-146)  | 15K (1-131)  |
|-----|--------------|--------------|
| Asp | 20.9 (20)    | 19.9 (20)    |
| Thr | 5.1 (5)      | 4.9 (5)      |
| Ser | 9.8 (11)     | 6.7 (9)      |
| Glu | 18.5 (18)    | 15.1 (16)    |
| Pro | * (2)        | * (2)        |
| Gly | 5.9 (5)      | 5.6 (4)      |
| Ala | 8.2 (8)      | 7.3 (7)      |
| Cys | * (2)        | * (2)        |
| Val | 9.1 (8)      | 9.1 (8)      |
| Met | 4.7 (4)      | 3.8 (3)      |
| Ile | 7.2 (7)      | 6.6 (7)      |
| Leu | 10.5 (10)    | 9.6 (9)      |
| Tyr | 5.5 (5)      | 4.8 (5)      |
| Phe | 10.3 (10)    | 8.2 (9)      |
| His | 1.8 (2)      | 2.5 (2)      |
| Lys | 19.9 (20)    | 16.9 (19)    |
| Arg | 8.6 (8)      | 5.6 (3)      |
| Trp | * (1)        | * (1)        |

Figures in parenthesis indicate the predicted amount of residues from DNA sequence.

* Values not determined.

## Table 8

### CNBr C-terminal Peptides of 15K and 18K

|  | 15K | 18K |
|---|---|---|
| Thr | 0.9 (1) |  |
| Ser | 1.1 (1) | 0.84 (1) |
| Glu | 1.1 (1) | 1.1 (1) |
| Pro | * (1) |  |
| Gly | 1.5 (1) | 1.3 (1) |
| Ala | 2.4 (3) | 1.1 (1) |
| Leu | 1.0 (1) | 1.1 (1) |
| Phe |  | 1.0 (1) |
| Lys | 1.9 (2) | 2.8 (3) |
| Positions in sequence | 121-131 | 138-146 |

Figures in parenthesis indicate the predicted residue number from DNA sequence.

* not determined.

References

1. Gray, P. W., et al., Nature 295, 503—508 (1982).
2. Stahelin, T., et al., J. Biol. Chem. 256, 9750—9754 (1981).
3. Allen, G., Sequencing of Protein and Peptides, pp. 30—31 (1981), North-Holland Publishing Co., Amsterdam, New York.
4. Amber, R. P., Methods in Enzymology 11, 436—445 (1967).
5. Wolfe, R. A. and Stein, S., Modern Methods in Pharmacology, pp. 55—77 (1982), Alan R. Liss, Inc., New York, NY.
6. Stein, S. and Brink, L., Methods in Enzymology, 79, 20—25 (1981).
7. Hewick, R. M., Hunkapillar, M. W., Hodd, L. E. and Dreyer, W. I., J. Biol. Chem. 256,, 7990—7997 (1981).
8. Hawke, D., Yuan, P—M., and Shively, J. E., Anal. Biochem. 120, 302—311 (1982).
9. Laemmli, U. K., Nature 227, 680—685 (1970).
10. Rubinstein, S., Familletti, P. C., and Pestka, S., J. Virol. 37, 755—758 (1981).

## Claims

1. A method for extracting intact recombinant human immune interferon protein essentially free from proteolytic fragments thereof from transformed microorganisms containing this protein characterized in that the extraction from said transformed microorganisms is performed with a guanidinium salt.

2. The method of Claim 1 wherein the guanidinium salt is guanidine-HCl.

3. The method of Claim 1 wherein the transformed microorganisms are suspended in a solution of guanidine-HCl.

4. The method of Claim 3 wherein the guanidine-HCl solution is at least about 4M.

5. The method of Claim 1 wherein the intact mature recombinant human immune interferon protein has a molecular weight of about 18,000 daltons.

6. The method of Claim 1 or Claim 2 wherein the transformed microorganisms preparation is separated after being treated with the guanidine salt into a superntant fraction and a cell debris fraction.

## Patentansprüche

1. Eine Methode zum Extrahieren von intaktem rekombinantem Human-Immun-Interferon Protein, das im wesentlichen frei von proteolytischen Fragmenten davon ist, aus transformierten, dieses Protein enthaltenden Mikroorganismen, dadurch gekennzeichnet, dass die Extraktion von besagten transformierten Mikroorganismen mit einem Guandiniumsalz durchgeführt wird.

2. Die Methode gemäss Anspruch 1, worin das Guanidiniumsalz Guanidiniumhydrochlorid ist.

3. Die Methode gemäss Anspruch 1, worin die transformierten Mikroorganismen in einer Lösung von Guanidiniumhydrochlorid suspendiert werden.

4. Die Methode gemäss Anspruch 3, worin die Guanidiniumhydrochlorid-Lösung wenigstens ungefähr 4M ist.

5. Die Methode gemäss Anspruch 1, worin das intakte reife rekombinante Human-Immun-Inerferon Protein ein Molekulargewicht von ungefähr 18,000 Dalton hat.

6. Die Methode gemäss Anspruch 1 oder 2, worin die transformierte Mikroorganismenpräparation nach Behandlung mit dem Guanidiniumsalz in eine Ueberstandfraktion und eine Zelltrümmerfraktion getrennt wird.

## Revendications

1. Procédé d'extraction d'une protéine interféron immun humain recombinant intact, essentiellement exempte de ses fragments protéolytiques, à partir de microorganismes transformés contenant cette protéine, caractérisé en ce que l'extraction à partir desdits microorganismes transformés se fait au moyen d'un sel de guanidinium.

2. Procédé selon la revendication 1, dans lequel le sel de guanidinium est le chlorhydrate de guanidine.

3. Procédé selon la revendication 1, dans lequel les microorganismes transformés sont mis en suspension dans une solution de guanidine-HCl.

4. Procédé selon la revendication 3, dans lequel la solution de guanidine-HCl est au moins environ 4M.

5. Procédé selon la revendication 1, dans lequel la protéine interféron immun humain recombinant intact mature a une masse moléculaire d'environ 18 000 daltons.

6. Procédé selon la revendication 1 ou la revendication 2, dans lequel la préparation de microorganismes transformés est séparée, après avoir été traitée avec le sel de guanidine, en une fraction surnageante et une fraction de débris cellulaires.

# FIG. 1

Reducing SDS–PAGE
A   I 5K  STDS   I 8K

94K
68K
43K
30K
21K
14K

I   2   3

Non–Reducing SDS–PAGE
B I 5K  STDS   I 8K

94K
68K
43K
30K
21K
14K

I   2   3

# FIG. 2

Sequence Comparison Between DNA Predicted Sequence and the 15K and 18K rIFN-y

```
        1                                                                                    15
        Cys - Tyr - Cys - Gln - Asp - Pro - Tyr - Val - Lys - Glu - Ala - Glu - Asn - Leu - Lys
15K     →*     →     →*    →      →     →     →     →     →     →     →     →     →     →     →
18K     →*     →     →*    →      →     →     →     →     →     →     →     →     →     →     →


                                                                             •          30
        Lys - Tyr - Phe - Asn - Ala - Gly - His - Ser - Asp - Val - Ala - Asp - Asn - Gly - Thr
15K     →     →     →     →     →     →     →     →     →     →     →     →     →     →     →
18K     →     →     →     →     →     →     →     →     →     →     →

                              35                                                         120
        Leu - Phe - Leu - Gly - Ile -------------------------------------------------------- Met

15K     →     →


        121                                                             131                    135
        Ala - Glu - Leu - Ser - Pro - Ala - Ala - Lys - Thr - Gly - Lys ------------------------
15K     →     →     →     →     →     →     →     →     →     →     →||

                                                                        146
              Met - Leu - Phe - Arg - Gly - Arg - Arg - Ala - Ser - Gln
18K           →     →     →     →     →     →     →     →     →     →||
```

Figure 2 — Footnotes

The arrow (→) indicates the residue identified by sequence determination
The star ( * ) indicates the cysteine residue identified in the form of C14 carboxymethylated cysteine.
Vertical bars ( I I ) indicates the C—terminal end of the recombinant protein.

# FIG. 3